# EUROPEAN PATENT SPECIFICATION

(11) **EP 0 992 256 B1**
(45) Date of publication and mention of the grant of the patent: **26.04.2006**
(21) Application number: 99119147.9
(22) Date of filing: 05.10.1999
(51) Int. Cl.: A61M 1/38

(54) **Blood component collection apparatus**
Vorrichtung zur Entnahme von Blutkomponenten
Appareil pour collecter des composants du sang

(30) Priority: 05.10.1998 JP 29913598
(43) Date of publication of application: 12.04.2000
(73) Proprietor: TERUMO KABUSHIKI KAISHA, Tokyo 151 (JP)
(72) Inventor: Suzuki, Atsushi, Fujinomiya-shi, Shizuoka (JP)
(74) Representative: Casalonga, Axel

(56) References cited:
- EP-A- 0 885 619
- WO-A-94/25086
- WO-A-96/40399

## Description

### BACKGROUND OF THE INVENTION

The present invention relates to a blood component collection apparatus for separating blood into predetermined blood components.

To effectively utilize collected blood and reduce donor's burden, the collected blood is separated into various components centrifugally and components required for a donee are collected, whereas remaining components are returned to the donor.

In obtaining a platelet product in this blood component collection process, the blood donated from the donor is introduced to a blood component collection circuit to separate it into four components of plasma, white cells, platelets, and red cells by a centrifugal separator called a centrifugal bowl installed in the blood component collection circuit. Then, the platelets are collected in a container to produce a platelet product, whereas the remaining components, namely, the plasma, the white cells, and the red cells are returned to the donor.

As methods of collecting the platelets, first and second methods are disclosed in WO 94/25086 (Japanese Patent Application Laid-Open No. 8-509403). In the first method, the whole blood is fed to the centrifugal bowl, with a dilute solution being added thereto at a predetermined flow rate. In the second method, the whole blood is sent to the centrifugal bowl to separate it centrifugally into a low-density component, an intermediate-density component, and a high-density component. After the low-density component is retrieved in a first container, switch-over of circuits are made to circulate the low-density component at a first flow rate (fixed rate) and widen the intermediate-density component region in the centrifugal bowl. The intermediate-density component is taken out from the centrifugal bowl while the low-density component is re-circulating at a second flow rate (accelerated rate).

The first method, however, requires three pumps, namely, an ACD pump, a blood collection/return pump, and a circulation pump. Thus, the apparatus for the first method is large. In the second method, the platelets can be collected with two pumps. Thus, the apparatus for the second method is smaller than the apparatus for the first method. Nevertheless, the collected platelets include many white cells. There are demands for supply of a platelet-containing solution that can be produced as a platelet product containing a possible fewest number of white cells.

Accordingly, the object of the present invention is to provide a compact blood component collection apparatus capable of producing a high-concentration platelet-containing solution containing a small number of white cells at a high platelet collection efficiency.

### SUMMARY OF THE INVENTION

The object of this invention is to provide a blood component collection apparatus comprising a blood component collection circuit including: a centrifugal separator having a rotor, an internal blood storage space, an inlet and an outlet communicating with said blood storage space and centrifugally separating blood introduced thereinto through said inlet by a rotation of said rotor; a first line for connecting a connecting portion of a blood collection needle or a blood collection instrument and said inlet of said centrifugal separator with each other, a second line connected to said outlet of said centrifugal separator; a third line connected to said first line, for injecting an anticoagulant to said blood; a plasma collection bag having a first tube connected with said first line and a second tube connected with said second line; and a platelet collection bag connected with said second line; said blood component collection apparatus further comprising: a centrifugal separator drive unit for rotating said rotor of said centrifugal separator; a first liquid supply pump which is used for said first line and located at a position of said first line between said centrifugal separator and a connecting portion for connecting said first tube to said first line; a second liquid supply pump which is used for said third line; a plurality of flow path shutter means for opening/closing flow paths of said blood component collection circuit; and a controller for controlling said centrifugal separator drive unit, said first liquid supply pump, said second liquid supply pump, and a plurality of said flow path shutter means; wherein said controller controls said centrifugal separator drive unit, said first liquid supply pump, said second liquid supply pump, and a plurality of said flow path shutter means to execute a platelet collection operation including: a plasma collection/constant-speed circulation step including a first plasma collection step of collecting anticoagulant-added blood by starting said first and second liquid supply pumps and collecting a predetermined amount of plasma into said plasma collection bag by activating said centrifugal separator drive unit; and a constant-speed plasma circulation step which is executed after said execution of said first plasma collection step terminates to temporarily suspend a blood collection and circulate said plasma contained in said plasma collection bag to said centrifugal separator at a constant speed by activating said centrifugal separator drive unit; a plasma collection/acceleration circulation step including a second plasma collection step which is executed after said execution of said plasma collection/constant-speed circulation step terminates to collect said anticoagulant-added blood by starting said first and second liquid supply pumps and collect said plasma by activating said centrifugal separator drive unit; and an acceleration plasma circulation step which is executed after said execution of said second plasma collection step is executed to temporarily suspend said blood collection and circulate said plasma contained in said plasma collection bag to said centrifugal separator at an accelerated speed by activating said centrifugal separator drive unit, a platelet collection step which is executed alter said execution of said plasma collection/acceleration circulation step terminates to flow out platelets from said centrifugal separator and collect said platelets into said platelet collection bag by accelerating a plasma circulation rate by means of said first liquid supply pump, and a blood return step which is executed after said execution of said platelet collection step terminates to return said blood inside said centrifugal separator to a donor.

### BRIEF DESCRIPTION OF THE DRAWINGS

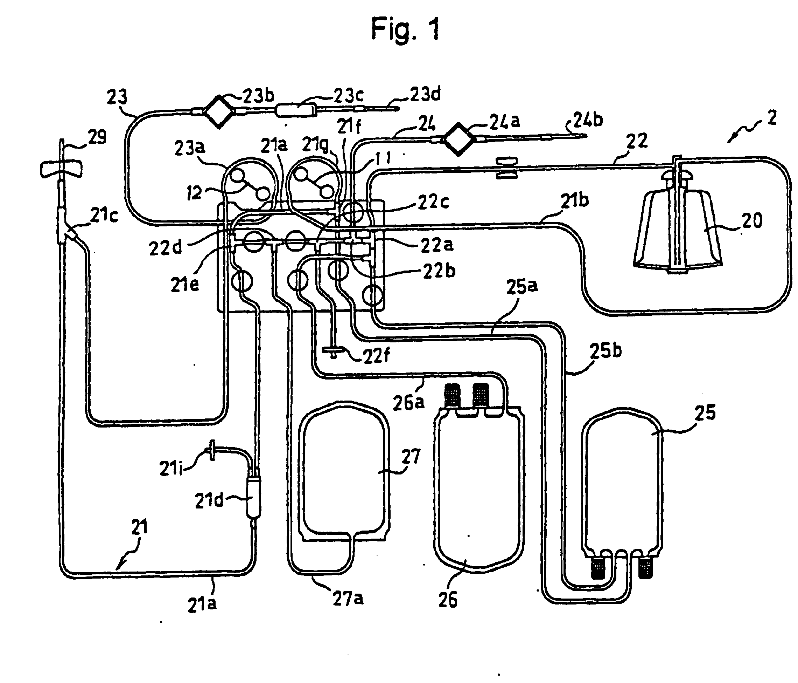

Fig. 1 is a schematic drawing of an example of the construction of a blood component collection circuit used in a blood component collection apparatus according to the present invention.
Fig. 2 is a plan view showing a cassette housing of the blood component collection circuit shown in Fig. 1.
Fig. 3 is a partially cutaway sectional view showing the state in which a drive unit is mounted on a centrifugal separator used in a blood component collection circuit.
Fig. 4 is a schematic drawing of a blood component collection apparatus according to an embodiment of the present invention having a blood component collection circuit mounted thereon.
Fig. 5 is a flowchart for describing the operation of the blood component collection apparatus according to the present invention.
Fig. 6 is a flowchart for describing the operation of the blood component collection apparatus according to the present invention.
Fig. 7 is the flowchart for describing the operation of a blood component collection apparatus according to the present invention.
Fig. 8 is a flowchart for describing the operation of the blood component collection apparatus according to the present invention.
Fig. 9 is a flowchart for describing the operation of the blood component collection apparatus according to the present invention.
Fig. 10 is a flowchart for describing the operation of the blood component collection apparatus according to the present invention.

### DETAILED DESCRIPTION OF REPRESENTATIVE EMBODIMENTS

A blood component collection apparatus according to the present invention will be described with reference to embodiments shown in the accompanying drawings.

A blood component collection apparatus 1 according to the present invention has a blood component collection circuit 2 including a centrifugal separator 20 having a rotor 142, an internal blood storage space and an inlet and an outlet both communicating with the internal blood storage space and centrifugally separating blood introduced into the internal blood storage space through the inlet by a rotation of the rotor 142; a first line 21 for connecting a connector of a blood collection needle 29 or a blood pool and the inlet of the centrifugal separator 20 with each other; a second line 22 connected to the outlet of the centrifugal separator 20; a third line 23 connected to the first line 21 and injecting an anticoagulant to the blood; a plasma collection bag 25 having a first tube 25a connected with the first line 21 and a second tube 25b connected with the second line 22; and a platelet collection bag 26 connected with the second line 22.

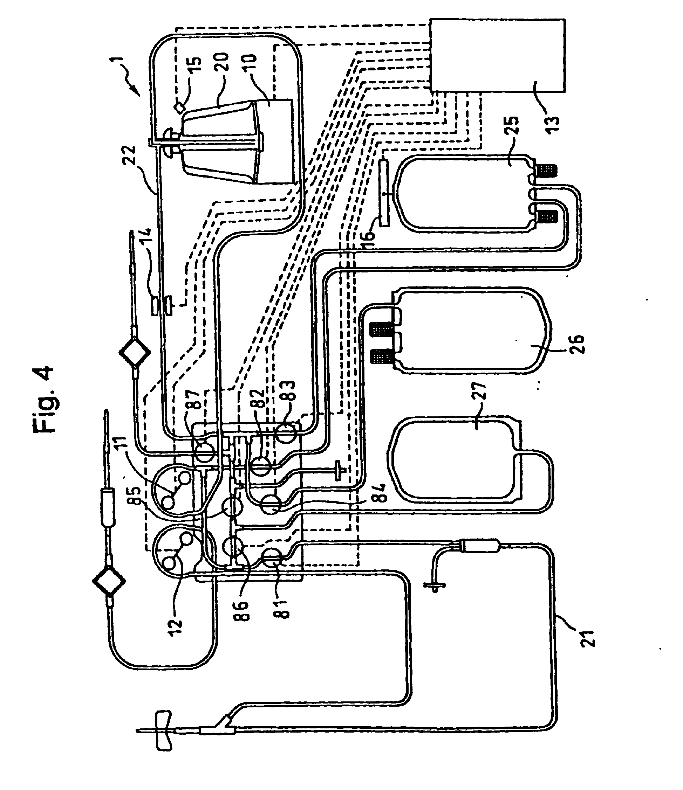

Referring to Fig. 4 in particular, the blood component collection apparatus 1 has a centrifugal separator drive unit 10 for rotating the rotor 142 of the centrifugal separator 20; a first liquid supply pump 11 to be used individually for the first line 21; a second liquid supply pump 12 to be used individually for the third line 23; a plurality of flow path shutter means 81, 82, 83, 84, 85, 86, and 87 for opening/closing flow paths of the blood component collection circuit 2, and a controller 13 for controlling the centrifugal separator drive unit 10, the first liquid supply pump 11, the second liquid supply pump 12, and a plurality of the flow path shutter means 81, 82, 83, 84, 85, 86, and 87.

The blood component collection circuit 2 will be described in detail below.

The blood component collection circuit 2 is used to collect a blood component, more specifically, platelets. The blood component collection circuit 2 includes a blood collector such as a blood collection needle 29 or a connector (connector of blood collector) connected with the blood collection needle 29 or with a blood collector having a blood pool connector; the first line 21 (blood collection/blood return line) connecting the blood collection needle 29 or the connector of the blood collector and the inlet of the centrifugal separator 20 with each other and having a first pump tube 21g; the second line 22 for connecting the outlet of the centrifugal separator 20 and the first line 21 with each other; the third line (anticoagulant injection line) 23 connected wit a position, of the first line 21, near the blood collection needle 29 and having a second pump tube 23a; a plasma collection bag 25 having the first tube 25a connected with a position, of the first line 21, located between the blood collection needle 29 and the first pump tube 21g and the second tube 25b connected with the second line 22; the platelet collection bag 26 having a third tube 26a connected with the second line 22; a buffy coat collection bag 27 having a fourth tube 27a connected with the second line 22; and a fourth line 24 connected with the second line 22 and injecting liquid (physiological salt solution). Instead of the blood collection needle 29, the blood component collection circuit 2 may employ a connector (such as a metal or plastic needle) to be connected with a blood pool such as a blood bag.

A known metal needle is used as the blood collection needle 29. The first line 21 includes a blood collection needle-side part 21a with which the blood collection needle 29 is connected and a centrifugal separator-side part 21b with which the inlet of the centrifugal separator 20 is connected. The blood collection needle-side part 21a is formed of a plurality of soft plastic tubes. The blood collection needle-side part 21a has the following elements arranged as viewed from the side of the blood collection needle 29: a branching connector 21c for connecting the blood collection needle-side part 21a and the third line 23 with each other; a chamber 21d for removing bubbles and micro-aggregates; a branching connector 21e for connecting the blood collection needle-side part 21a and the second line 22 with each other; and a branching connector 21f for connecting the first line 21 and the first tube 25a of the plasma collection bag 25 with each other. The chamber 21d is connected to an air-permeable and germ-blocking filter 21i. The centrifugal separator-side part 21b is connected with the branching connector 21f connected with the first tube 25a and has the first pump tube 21g formed in the vicinity of the branching connector 21f.

The second line 22 connecting the outlet of the centrifugal separator 20 and the first line 21 with each other has an end thereof connected to the outlet of the centrifugal separator 20 and the other end thereof connected to the branching connector 21e for connecting the blood collection needle-side part 21a and the second line 22 with each other. The second line 22 has the following elements arranged as viewed from the side of the centrifugal separator 20: a branching connector 22a for connecting the second line 22 with the second tube 25b of the plasma collection bag 25 and with the third tube 26a of the platelet collection bag 26; a branching connector 22b for connecting the second line 22 with the fourth line 24; a branching connector 22c for connecting the second line 22 with a tube having a bubble-removing filter 22f; and a branching connector 22d for connecting the second line 22 with the fourth tube 27a connected with the buffy coat collection bag 27.

The third line 23 is connected with the branching connector 21c having an end thereof connected to the first line 21. The third line 23 has the following elements arranged as viewed from the connector 21c: a pump tube 23a; a foreign matter removing filter 23b; a bubble removing chamber 23c; and an anticoagulant container-connecting needle 23d.

One end of the fourth line 24 is connected with the branching connector 22b for connecting the second line 22 and the fourth line 24 with each other. The fourth line 24 has the following elements arranged as viewed from the connector 22b: a foreign matter-removing filter 24a and a physiological salt solution container connecting needle 24b.

The plasma collection bag 25 includes the first tube 25a connected with the branching connector 21f located between the blood collection needle 29 and the pump tube 21g of the first line 21; and the second tube 25b connected with the branching connector 22a of the second line 22. The platelet collection bag 26 includes the third tube 26a connected with the branching connector 22a of the second line 22. The buffy coat collection bag 27 includes the fourth tube 27a connected with the branching connector 22d of the second line 22.

The tubes and the pump tubes used to form the first to fourth lines and the tubes connected to the bags as described above are composed of such materials as polyvinyl chloride, polyethylene, polypropylene, polyester such as PET or PBT, ethylene-vinyl acetate copolymer, polyurethane, polyester elastomer, thermoplastic elastomer such as styrene-butadiene-styrene copolymer. Of all these component materials, polyvinyl chloride is the most favorable. If all the tubes are made of polyvinyl chloride, they have sufficient flexibility and plasticity for easy handling. Also, this material is suitably used to cope with clogging caused by a clamp and the like. Component materials similar to these tube materials can also be used to form the branching connectors. The pump tubes strong enough to bear a pressure applied thereto by roller pumps 11, 12 are used in the embodiment.

Each of the plasma collection bag 25, the platelet collection bag 26, and the buffy coat collection bag 27 is composed of layers of flexible plastic sheets having the peripheral edges thereof fusion bonded (thermally or with high frequencies) or adhered. Soft polyvinyl chloride is the most favorable material for forming the bags 25, 26, and 27. Plasticizers used for the soft polyvinyl chloride include, for example, di-(ethylhexyl) phthalate (DEHP), di-(n-decyl) phthalate (DnDP) and the like. The content of these plasticizers preferably ranges from approximately 30 to 70 parts by weight for 100 parts by weight of the polyvinyl chloride.

Other sheet materials that can be used to form the bags 25, 26, and 27 are polyolefin, namely, a polymer formed by polymerizing or copolymerizing an olefin or di-olefin of ethylene, propylene, butadiene, isoprene or the like. More specifically, polyethylene, polypropylene, ethylene-vinyl acetate copolymer (EVA), polymer blend of EVA and various thermoplastic elastomers, etc. or an arbitrary combination thereof can be used. Still other applicable materials include polyester such as polyethylene terephthalate (PET), polybutylene terephthalate (PBT), poly-1, 4-cyclohexanedimethyl terephthalate (PCHT) and polyvinylidene chloride.

It is preferable to use a sheet material superior in gas permeability for the platelet collection bag 26 for assuring an improved platelet shelf life. Such sheet materials include the above-mentioned polyolefin or DnDP plasticized polyvinyl chloride, etc. Alternatively, comparatively thin (for example, about 0.1 to 0.5 mm thick, or especially, about 0.1 to 0.3 mm thick) materials can be preferably used. The platelet collection bag can be preliminarily filled with a platelet preservation liquid such as physiological salt solution, GAC, PAS, PSM-1.

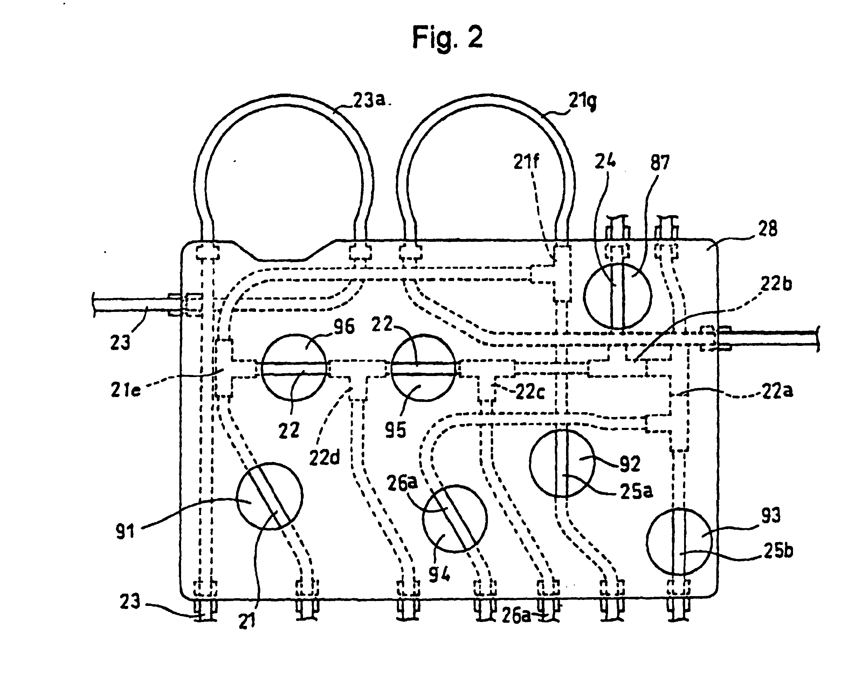

Essential portions of the blood component collection circuit 2 are housed in a cassette, as shown in Fig. 2. The blood component collection circuit 2 includes a cassette housing 28 partially accommodating or partially holding all the lines (first, second, third, and fourth lines) and all the tubes (first, second, third, and fourth tubes). In other words, they are partially fixed to the cassette housing 28. The cassette housing 28 holds both ends of the first pump tube 21g and the second pump tube 23a fixed thereto. The pump tubes 21g, 23a project from the cassette housing 28 in the shape of a loop in correspondence to that of the roller pumps 11, 12, respectively. Therefore, the first and the second pump tubes 21g, 23a can be readily mounted on the roller pumps 11, 12, respectively.

Further, the cassette housing 28 has a plurality of openings located therein. Specifically, located in the cassette housing 28 are a first opening 91 that exposes the portion of the first line 21 between the blood collection needle 29 and the pump tube 21g and can be penetrated by the first flow path shutter means 81 of the blood component collection apparatus 1; a second opening 92 that exposes the first tube 25a of the plasma collection bag 25 and can be penetrated by the second flow path shutter means 82 of the blood component collection apparatus 1; a third opening 93 that exposes the second tube 25b of the plasma collection bag 25 and can be penetrated by the third flow path shutter means 83 of the blood component collection apparatus 1; a fourth opening 94 that exposes the third tube of the platelet collection bag 26 and can be penetrated by the fourth flow path shutter means 84 of the blood component collection apparatus 1; a fifth opening 95 that exposes the portion (upstream side) of the second line 22 located between the centrifugal separator (connector 22c) 20 and the connector 22d for connecting the fourth tube 27a of the buffy coat collection bag 27 and the second line 22 with each other and can be penetrated by the fifth flow path shutter means 85 of the blood component collection apparatus 1; a sixth opening 96 that exposes the portion of the second line 22 located between the connector 21e for connecting the first line 21 and the second line 22 with each other and the connector 22d for connecting the fourth tube 27a of the buffy coat collection bag 27 and the second line 22 with each other and can be penetrated by the sixth flow path shutter means 86 of the blood component collection apparatus 1; and a seventh opening 97 that exposes the fourth line 24 and can be penetrated by the seventh flow path shutter means 87 of the blood component collection apparatus 1.

The above-mentioned branching connectors are fixed to the inner surface of the cassette housing 28. In the vicinity of the side surface of the cassette housing 28, there are provided reinforcing tubes for holding the lines and tubes projecting from the side surface of the cassette housing 28 and preventing them from being bent. The cassette housing 28 is box-shaped and contains parts indicated with dashed lines of Fig. 2. The cassette housing 28 is formed of synthetic resin having a certain degree of rigidity.

The blood component collection apparatus 1 includes a cassette housing-mounting portion (not shown). When the cassette housing 28 is mounted on the cassette housing-mounting portion of the blood component collection apparatus 1, the lines and the tubes exposed from the openings of the cassette housing 28 are automatically mounted on the corresponding flow path shutter means, respectively. Thus, the blood component collection circuit 2 can be easily mounted on the blood component collection apparatus 1 and rapid preparations for collecting blood components can be accomplished. The blood component collection apparatus 1 has two pumps proximately to the cassette housing-mounting portion. Thus, the pump tubes exposed from the cassette housing 28 can be easily mounted on the pumps.

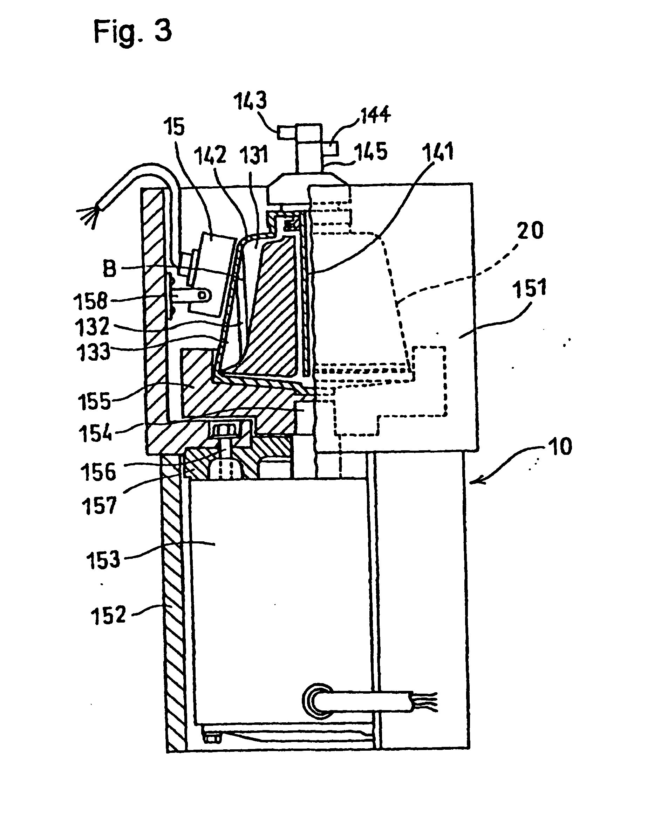

The centrifugal separator 20, a so-called centrifugal bowl, is mounted on the blood component collection circuit 2 to separate blood into components by a centrifugal force. As shown in Fig. 3, the centrifugal separator 20 includes a tubular member 141 extending vertically and having an inlet 143 formed at the upper end thereof and a hollow rotor 142 sealed to prevent liquid from flowing thereinto from an upper portion 145 of the centrifugal separator 20 and rotating around the tubular member 141. The rotor 142 has a flow path (blood storage space) formed along the bottom and the inner peripheral surface thereof. An outlet 144 is so formed as to communicate with the upper portion of the flow path. The volume of the rotor 142 ranges from 100 to 350 ml.

The rotor 142 is rotated under predetermined centrifugal conditions (rotational speed and rotation time) set by the rotor (centrifugal separator) drive unit 10 of the blood component collection apparatus 1. Based on the centrifugal conditions, patterns (for example, the number of blood components to be separated) of blood separation to be made in the rotor 142 can be set. As shown in Fig. 3, according to the embodiment, the centrifugal conditions are set such that the blood is separated into a plasma layer (inner layer) 131, a buffy coat layer (intermediate layer) 132, and a red cell layer (outer layer) 133 laminated in the flow path of the rotor 142.

Referring to Fig. 4, the blood component collection apparatus 1 according to the present invention will be described.

The blood component collection apparatus 1 indudes the centrifugal separator drive unit 10 for rotating the rotor 142 of the centrifugal separator 20; the first liquid supply pump 11 to be used individually for the first line 21; the second liquid supply pump 12 to be used individually for the third line 23; a plurality of the flow path shutter means 81, 82, 83, 84, 85, 86, and 87 for opening/closing the flow paths of the blood component collection circuit 2; and the controller 13 for controlling the centrifugal separator drive unit 10, the first liquid supply pump 11, the second liquid supply pump 12, and the flow path shutter means 81, 82, 83, 84, 85, 86, and 87. The blood component collection apparatus 1 further includes a turbidity sensor 14 mounted on the portion, of the second line 22, located between the centrifugal separator 20 and the connector 22a for connecting the second tube 25b with the second line 22; an optical sensor 15 mounted above the centrifugal separator 20; and a weight sensor 16 for detecting the weight of the plasma collection bag 25.

The controller 13 includes two pump controllers (not shown) one of which is used individually for the first liquid supply pump 11 and the other of which is used individually for the second liquid supply pump 12. A control mechanism of the controller 13 is electrically connected to the first liquid supply pump 11 through the one pump controller and to the second liquid supply pump 12 through the other pump controller. A drive controller of the centrifugal separator drive unit 10 is electrically connected with the controller 13. The flow path shutter means 81, 82, 83, 84, 85, 86, and 87 are all connected with the controller 13 for controlling the opening/closing thereof. The turbidity sensor 14, the optical sensor 15 installed above the centrifugal separator 20, and the weight sensor 16 for detecting the weight of the plasma collection bag 25 are electrically connected with the controller 13 to which signals are transmitted from these sensors. The control mechanism of the controller 13 is constructed of a microcomputer. Detection signals outputted from the weight sensor 16, the optical sensor 15, and the turbidity sensor 14 are applied to the controller 13. The controller 13 controls the start and stop of the rotation and rotational direction (forward/backward) of each pump, based on the signals applied thereto from the turbidity sensor 14, the optical sensor 15, and the weight sensor 16. The controller 13 also controls the opening/closing of the flow path shutter means 81, 82, 83, 84, 85, 86, and 87 and the centrifugal separator drive unit 10.

The first flow path shutter means 81 is used to open/close the portion of the first line 21 located between the blood collection needle 29 and the pump tube 21g. The second flow path shutter means 82 is used to open/close the first tube 25a of the plasma collection bag 25. The third flow path shutter means 83 is used to open/close the second tube 25b of the plasma collection bag 25. The fourth flow path shutter means 84 is used to open/dose the third tube 26a of the platelet collection bag 26. The fifth flow path shutter means 85 is used to open/close the portion of the second line 22 located between the centrifugal separator 20 and the connector 22d for connecting the second line 22 with the fourth tube 27a of the buffy coat collection bag 27. The sixth flow path shutter means 86 is used to open/close the portion of the second line 22 located downstream from the connector 21e for connecting the first line 21 and the second line 22 with each other to the connector 22d for connecting the fourth tube 27a of the buffy coat collection bag 27 and the second line 22 with each other. The seventh flow path shutter means 87 is used to open/close the fourth line 24. Each of the flow path shutter means 81 through 87 has an insertion portion on which the lines or the tubes are installed. The insertion portion has a clamp to be operated by a drive source such as a solenoid, an electric motor, and a hydraulic or pneumatic cylinder. An electromagnetic clamp to be operated by the solenoid is preferable. The clamp of each of the flow path shutter means 81 through 87 is operated based on a signal transmitted thereto from the controller 13.

As shown in Fig. 3, the centrifugal separator drive unit 10 includes a rotor drive unit housing 151 accommodating the centrifugal separator 20, a pedestal 152, a motor 153 constituting the drive source, and a disk-shaped rest 155 for holding the centrifugal separator 20. The housing 151 is fixedly mounted on the pedestal 152. The motor 153 is fixed to the lower surface of the housing 151 with a bolt 156 via a spacer 157. The upper end of the rotary shaft 154 of the motor 153 is fitted in the rest 155 such that the rest 155 rotates coaxially and integrally with the rotational shaft 154. The upper portion of the rest 155 has a recess formed therein into which the bottom of the rotor 142 is fitted. The upper portion 145 of the centrifugal separator 20 is fixed to the housing 151 with a fixing member not shown. Once the motor 153 for the rotor drive unit 10 is started, the rest 155 and the rotor 142 fixed thereto are rotated at a rotational speed ranging from 3000 to 6000 rpm.

The rotor drive unit housing 151 has the optical sensor 15 fixedly mounted on an inner wall thereof with a mounting member 158. The optical sensor 15 optically detects the boundaries between separated blood components (for example, the boundary B between the plasma layer 131 and the buffy coat layer 132 and the boundary between the buffy coat layer 132 and the red cell layer 133) in the centrifugal separator 20. The optical sensor 15 is of a type capable of vertically scanning the peripheral surface of the rotor 142. The optical sensor 15 indudes a light source for emitting a light beam to a shoulder portion of the centrifugal separator 20 and a light-receiving part for receiving a light beam reflected by the centrifugal separator 20 and returned therefrom. That is, a light emitting element such as an LED or laser and a light-receiving element are arrayed in a row. A light beam emitted by the light emitting element and reflected by a blood component is received by the light-receiving element, and the amount of the received light is photoelectrically converted. Because the intensity of the reflected light varies depending on the separated blood component (for example, the boundary B between the plasma layer 131 and the buffy coat layer 132), a position of the light-receiving element at which the amount of received light has changed is detected as the corresponding position of the boundary B. More specifically, the arrival of the buffy coat layer at a light passage portion is detected, based on the difference between the amount of received light at the time when the light passage portion of the centrifugal separator 20 is filled with a transparent liquid (plasma or water) and the amount of received light at the time when the light passage portion thereof is filled with the buffy coat layer. The detection position of the buffy coat layer is adjusted by changing a light passage position in the centrifugal separator 20. Normally, the detection of the light passage position is unchangeably set.

The turbidity sensor 14 detects the turbidity of a fluid flowing through the second line 22 and outputs a voltage corresponding to the detected turbidity. Specifically, the turbidity sensor 14 outputs a low voltage when the turbidity is high, while it outputs a high voltage when the turbidity is low.

As each of the first liquid supply pump 11 on which the pump tube 21g of the first line 21 is mounted and the second liquid supply pump 12 on which the pump tube 23a of the third line 23 is mounted, a roller pump or a peristaltic pump which is kept out of contact with the blood is preferably used. A pump that can supply the blood in either direction is used as the first liquid supply pump 11 (blood pump). A roller pump capable of rotating forward/ backward is used.

The controller 13 executes a plasma collection/constant-speed circulation step and then, a plasma collection/acceleration circulation step. More specifically, the plasma collection/constant-speed circulation step includes a first plasma collection step of collecting anticoagulant-added blood by starting the first and second liquid supply pumps 11, 12 and collecting a first predetermined amount of plasma into the plasma collection bag 25 by activating the centrifugal separator drive unit 10. The plasma collection/constant-speed circulation step also includes a constant-speed plasma circulation step of temporarily suspending the blood collection and circulating the plasma contained in the plasma collection bag 25 to the centrifugal separator 20 at a constant speed by activating the centrifugal separator drive unit 10. The plasma collection/acceleration circulation step indudes a second plasma collection step of collecting the anticoagulant-added blood by starting the first and second liquid supply pumps 11, 12 and collecting the plasma by activating the centrifugal separator drive unit 10 until a boundary sensor detects a predetermined position (for example, BC layer). The plasma collection/acceleration circulation step also includes an acceleration plasma circulation step of temporarily suspending the blood collection after the second plasma collection step terminates and circulating the plasma contained in the plasma collection bag 25 to the centrifugal separator 20 at an accelerated speed by activating the centrifugal separator drive unit 10. In the second plasma collection step, the boundary sensor detects the boundary between blood components. Thus, detection of the weight of the plasma bag is not executed.

The controller 13 of the blood component collection apparatus 1 according to the embodiment controls the centrifugal separator drive unit 10, the first liquid supply pump 11, the second liquid supply pump 12, and a plurality of the flow path shutter means 81 through 87 to execute the platelet collection operation including the plasma collection/constant-speed circulation step and the plasma collection/acceleration circulation step twice.

Subsequent to the termination of the execution of a platelet collection step and prior to the start of the execution of a blood return step, the controller 13 of the blood component collection apparatus 1 executes a buffy coat-collection step of flowing out the buffy coat from the centrifugal separator 20 and collecting it into the buffy coat collection bag 27 by setting a plasma circulation rate higher than a final plasma circulation rate in the platelet collection step by means of the first liquid supply pump 11. The buffy coat collection step is not limited to the above-mentioned method. As an example, the buffy coat collection step may be executed by maintaining the plasma circulation rate at the final plasma circulation rate in the platelet collection step by means of the first liquid supply pump 11 and decreasing the rotational speed of the rotor 142 of the centrifugal separator 20. As another example, the buffy coat collection step may be executed by setting the plasma circulation rate higher than the final plasma circulation rate in the platelet collection step by means of the first liquid supply pump 11 and decreasing the rotational speed of the rotor 142 of the centrifugal separator 20.

After the execution of the buffy coat collection step terminates and before the execution of the subsequent blood collection step starts, the controller 13 controls the first liquid supply pump 11 and a plurality of the flow path shutter means 81 through 87 to execute the buffy coat return step of returning the collected buffy coat to the centrifugal separator 20.

Specifically, an anticoagulant is added to the whole blood at a predetermined ratio (1/8 to 1/20, specifically, 1/10 of the whole blood). The anticoagulant-added blood is fed to the centrifugal separator 20 through the first line 21 at a predetermined rate (250 ml/min or less, preferably, 150 to 40 ml/min or less, or specifically, 60 ml/min or less). The centrifugal separator 20 is rotated at a predetermined rotational speed (3000 to 6000 rpm, or preferably, 4700 to 4800 rpm) to separate the blood into the plasma, the buffy coat, and the red cells. The plasma that has overflowed the centrifugal separator 20 is collected in the plasma collection bag 25. At a time point when a predetermined amount (10 to 150 ml, or preferably 20 to 30 ml) of the plasma is collected, the blood supply is stopped. Then, the plasma is returned to the centrifugal separator 20 through the first and second lines 21, 22 under predetermined conditions (at a rate higher than that in the blood collection, namely, 60 to 250 ml/min for 10 to 90 seconds, or specifically, 200 ml/min for 30 seconds in the first circulation). That is, a constant-speed plasma circulation is executed.

Then, the anticoagulant is added again to the whole blood at the predetermined ratio (1/8 to 1/20, specifically, 1/10 of the whole blood). The anticoagulant-added blood is fed to the centrifugal separator 20 through the first line 21 at the predetermined rate (250 ml/min or less, preferably, 150 to 40 ml/min or less, specifically, 60 ml/min or less). The centrifugal separator 20 is rotated at the predetermined rotational speed (3000 to 6000 rpm, preferably, 4700 to 4800 rpm) to separate the blood into the plasma, the buffy coat, and the red cells. When the position of the boundary of blood cells inside the centrifugal separator 20 is detected by the buffy coat boundary sensor, the blood supply is stopped. Then, the plasma is returned to the centrifugal separator 20 through the first and second lines 21, 22 under predetermined conditions (initial speed: 60 to 250 ml/min, final arrival speed (set speed) 150 - 250 ml/min, acceleration condition: increase of 2 - 10 ml/min, and circulation time period: 10 - 90 seconds). That is, an acceleration plasma circulation is executed.

After the whole anticoagulant-added blood is fed again under predetermined conditions (collection amount of blood: 0 to 2500/Hct% [ml], specifically, 400 to 1000/Hct% [ml]), the plasma is returned to the centrifugal separator 20 through the first and second lines 21, 22 under the predetermined conditions. The acceleration rate is increased stepwise under predetermined conditions (stepwise acceleration ranging from 0.1 to 99 ml/min/sec, or specifically, 2-10 ml/min/sec) until the platelet collection rate (60 to 250 ml/min, or actually, 200 ml/min) is attained. The platelets released from the centrifugal separator 20 are collected in the platelet collection bag 26. Then, in the blood component collection apparatus 1, the blood circulation rate (at 60 to 250 ml/min., specifically, 200 ml/min) is maintained while reducing the rotational speed of the centrifugal separator 20 (by 100 to 300 rpm, preferably by 4500 to 4600 rpm). In this way, the released buffy coat is collected and supplied to the centrifugal separator 20 prior to the start of the next blood collection cycle. The buffy coat may be collected by increasing the blood circulation rate to a predetermined one (not less than the platelet collection rate, preferably 60 to 250 ml/min., specifically, 205 ml/min.) after the platelets are collected.

The blood component collection process (first platelet collection operation) to be executed by the blood component collection apparatus 1 according to the embodiment will be described with reference to flowcharts of Figs. 4, 5 to 10. In the embodiment, the platelet collection operation is repeated twice, and after the execution of each platelet collection step other than the last one terminates and prior to the start of the execution of the blood return step, the buffy coat collection step is executed. Prior to the start of the execution of the next blood collection step, the buffy coat return step is performed to return the buffy coat to the centrifugal separator 20.

First, the third line 23 and the blood collection needle 29 are primed with the anticoagulant and then the blood collection needle 29 is stuck on the donor.

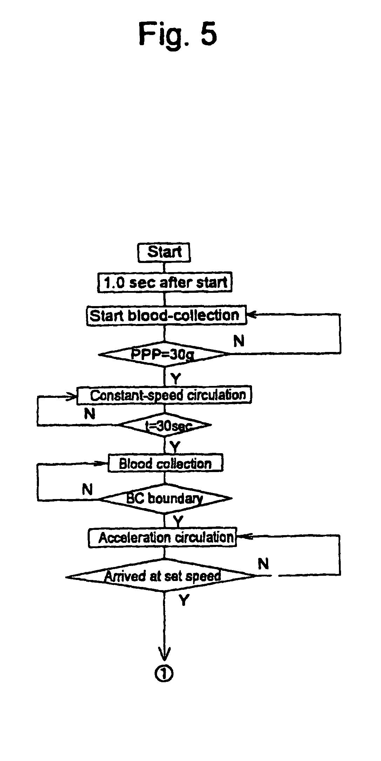

As shown in Fig. 5, the controller 13 executes the first plasma collection step of collecting the anticoagulant-added blood by starting the first and second liquid supply pumps 11, 12 and collecting the first predetermined amount of the plasma into the plasma collection bag 25 by activating the centrifugal separator drive unit 10.

When the first blood collection starts, the blood pump 11 is rotated at a predetermined rate (for example, 60 ml/min). At this time, a second pump constituting the anticoagulant pump supplies the anticoagulant (for example, ACD-A solution) at a predetermined rate (for example, 1/10 of the blood pump rate). The blood drawn from the donor is mixed with the ACD-A solution, flows through the first line 21, the chamber, and the first flow path shutter means 81, and flows into the centrifugal separator 20. At the above time point, the sixth flow path shutter means 86, the fifth flow path shutter means 85, the second flow path shutter means 82, the third flow path shutter means 83, and the seventh flow path shutter means 87 are closed. Meanwhile the first flow path shutter means 81 and the fourth flow path shutter means 84 are open. When the ACD-A solution-added blood is fed to the centrifugal separator 20, sterilized air that has stayed in the centrifugal separator 20 flows into the platelet collection bag 26 through the second line 22 and the flow path shutter means 84. Simultaneously with the start of the blood collection, the centrifugal separator 20 starts rotating at a predetermined speed (for example, 4800 rpm) while it is being supplied with the ACD-A solution-added blood. In the centrifugal separator 20, therefore, the blood is centrifugally separated into a plasma layer (inner layer), a buffy coat layer (BC layer, intermediate layer), and a red cell layer (outer layer). When the ACD-A solution-added blood (about 270 ml) is supplied in excess of the capacity of the centrifugal separator 20, the centrifugal separator 20 is completely filled with the blood, and the plasma overflows through the outlet of the centrifugal separator 20. The turbidity sensor 14 mounted on the second line 22 connected with the outlet of the centrifugal separator 20 detects that the fluid flowing through the second line 22 has changed from the air to the plasma. Upon receipt of the detection signal transmitted from the turbidity sensor 14, the controller 13 doses the fourth flow path shutter means 84 and opens the third flow path shutter means 83 to collect the plasma into the plasma collection bag 25. The weight of the plasma collection bag 25 is measured in advance by the weight sensor 16, and a signal indicating the measured weight is transmitted to the controller 13. Thus, when the weight of the plasma collected in the plasma collection bag 25 is increased to a predetermined amount (10 to 150 g, for example, 20 g), the controller 13 closes the first flow path shutter means 81 and opens the second flow path shutter means 82 to proceed to the constant-speed plasma circulation step.

In the constant-speed circulation step, the blood collection is temporarily suspended, and the centrifugal separator drive unit 10 is activated to circulate the plasma contained in the plasma collection bag 25 to the centrifugal separator 20 at a constant speed.

Upon start of the execution of the constant-speed circulation step, the controller 13 keeps the first flow path shutter means 81 closed and the second flow path shutter means 82 opened, stops the ACD-A pump 12, and starts the blood pump 11 at the predetermined rate (60 to 250 ml/min, for example, 200 ml/min). The plasma collected in the plasma collection bag 25 is fed to the centrifugal separator 20 rotating at 4800 rpm through the second flow path shutter means 82. At the same time, the plasma which has flowed from the centrifugal separator 20 flows into the plasma collection bag 25 through the turbidity sensor 14 and the third flow path shutter means 83. With the lapse of a predetermined length of time (10 to 90 sec, for example, 30 seconds) from the start of the constant-speed circulation step, the controller 13 closes the second flow path shutter means 82 and opens the first flow path shutter means 81 to proceed to the second plasma collection step. The first plasma circulation step is executed preferably at the flow rate of 60 ml/min or more for at least 10 seconds.

In the second plasma collection step, the first and second liquid supply pumps 11, 12 are started to collect the anticoagulant-added blood. Upon detection of the buffy coat layer in the centrifugal separator 20 because of an increase in the amount of the plasma in the platelet collection bag 26, a detection signal is transmitted from the optical sensor 15 to the controller 13. Upon receipt of the signal, the controller 13 closes the first flow path shutter means 81 and opens the second flow path shutter means 82 to proceed to the acceleration plasma circulation step.

More specifically, the first blood pump 11 is activated to collect the blood at a predetermined rate (for example, 60 ml/min). At this time, the second pump constituting the anticoagulant pump supplies the anticoagulant (for example, ACD-A solution) at a predetermined rate (for example, 1/10 of the rate of the blood pump). The blood drawn from the donor is mixed with the ACD-A solution. The ACD-A solution-added blood flows into the centrifugal separator 20 rotating at a predetermined rate (for example, 4800 rpm), and the plasma is collected in the plasma collection bag 25. Normally, upon detection of the buffy coat layer in the centrifugal separator 20 because of an increase in the amount of the plasma in the platelet collection bag 26, a detection signal is transmitted from the optical sensor 15 to the controller 13. Upon receipt of the signal, the controller 13 closes the first flow path shutter means 81 and opens the second flow path shutter means 82 to proceed to the acceleration plasma circulation step. In the second plasma collection step, the plasma is collected until the optical sensor 15 detects the buffy coat (BC boundary: boundary between plasma layer and buffy coat layer).

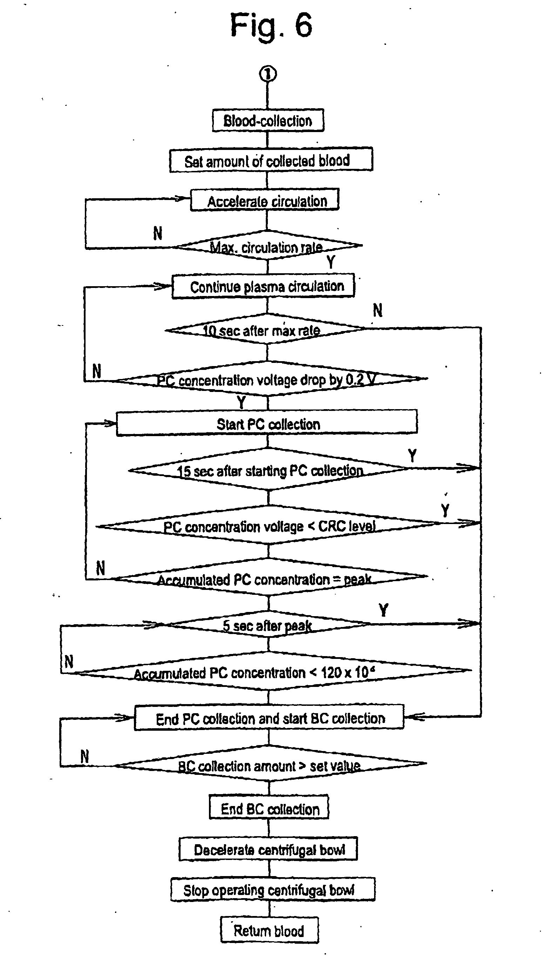

In the acceleration plasma circulation step, the blood collection is temporarily suspended and the centrifugal separator drive unit 10 is activated to circulate the plasma in the plasma collection bag 25 to the centrifugal separator 20. In the process, the blood pump operates at an initial rate, for example, at 60 ml/min lower than that in the constant-speed plasma circulation step and accelerated at 6.7 ml/min for 21 seconds until a final rate reaches 200 ml/min. Upon completion of the acceleration plasma circulation step, the process proceeds to ① of the flowchart of Fig. 6 showing a blood collection step for adjustment of boundary.

As shown in Fig. 6, the blood collection step for boundary adjustment can be regarded as a step of collecting a small amount of plasma. In this step, in order to keep the position of the buffy coat layer constant, irrespective of donors in the subsequent platelet collection step, the blood is collected in correspondence to a predetermined amount of red cells to be supplied. The supply amount of the red cells is defined as a value derived from dividing a collected amount of blood by the hematocrit value of the donor. Practically about 12 ml of the blood is collected. The blood collection starts by rotating the first liquid supply pump 11 at a predetermined rate (for example, 60 ml/min). At this time, the second pump constituting the anticoagulant pump also supplies the anticoagulant (such as the ACD-A solution) at a predetermined rate (for example, 1/10 of the blood pump rate). The blood collected from the donor is mixed with the ACD-A solution. The ACD-A solution-added blood flows into the centrifugal separator 20 rotating at a predetermined rate (for example, 4800 rpm), and a small amount of plasma is collected. Based on a set collection amount and a set pump rotation rate, the controller 13 computes a blood collection time, and terminates the blood collection when the computed blood collection time expires. Then the controller 13 closes the first flow path shutter means 81 and opens the second flow path shutter means 82 to proceed to the platelet collection step.

Upon termination of the acceleration plasma circulation step, the controller 13 executes the platelet collection step of flowing out platelets from the centrifugal separator 20 and collecting them into the platelet collection bag 26 by accelerating the plasma circulation rate by means of the first liquid supply pump 11. The platelet collection step is called an acceleration process. In this step, the controller 13 controls the rotational speed of the blood pump to increase it stepwise by 10 ml/min every predetermined time period (for example, every one second) from 60 ml/min to 200 ml/min. When the rotational speed of the blood pump reaches 200 ml/min, it is maintained until the platelet collection step terminates.

Upon start of the platelet collection step, the turbidity sensor 14 detects the turbidity of a liquid passing therethrough and outputs a voltage corresponding to a detected turbidity. The output signal of the turbidity sensor 14 is applied to the controller 13. When the rotational speed of the blood pump increases and reaches a rate ranging from 160 to 200 ml/min, platelets contained in the buffy coat layer that have stayed in the centrifugal separator 20 flow out therefrom. As a result of the release of the platelets, the turbidity of the liquid passing through the turbidity sensor 14 becomes high. At a time point when a voltage outputted from the turbidity sensor 14 drops by 0.2 V, the third flow path shutter means 83 is closed and the fourth flow path shutter means 84 is opened. Thus the platelet-rich plasma released from the centrifugal separator 20 is collected in the platelet collection bag 26. The controller 13 converts the voltage outputted from the turbidity sensor 14 into a platelet concentration to compute a platelet concentration in the platelet collection bag 26 during the platelet collection operation. The platelet concentration in the platelet collection bag 26 drops after reaching a maximum value. At a time point when it is detected that the maximum concentration has been attained, the execution of the platelet collection step finishes and the process proceeds to the buffy coat collection step.

Upon completion of the platelet collection step, the buffy coat collection step is executed. In this step, the controller 13 closes the fourth flow path shutter means 84 and opens the fifth flow path shutter means 85. The plasma in the plasma collection bag 25 is fed to the centrifugal separator 20 by the blood pump 11. At the same time, the liquid that has been released (the buffy coat layer that has flowed out) from the centrifugal separator 20 flows into the buffy coat collection bag 27. In the buffy coat collection step, the final rate of the rotational speed of the blood pump in the platelet collection step is maintained, and the rotational speed of the centrifugal separator 20 is decreased to 4600 rpm. In this manner, the buffy coat is released from the centrifugal separator 20 and collected in the buffy coat collection bag 27. At a time point when the amount of the collected buffy coat reaches a value computed based on the hematocrit value of the donor and the amount of collected platelet, the blood pump 11 is stopped and all the valves are closed. Thus the centrifugal separator 20 stops rotating, i.e., the execution of the buffy coat collection step terminates.

Then, the blood return step of returning the blood in the centrifugal separator 20 to the donor is executed. The controller 13 rotates the blood pump 11 in a reverse direction, opens the first flow path shutter means 81, and returns the red cell layer remaining in the centrifugal separator 20 to the donor through the first line 21.

Thus, the first (initial) platelet collection operation terminates.

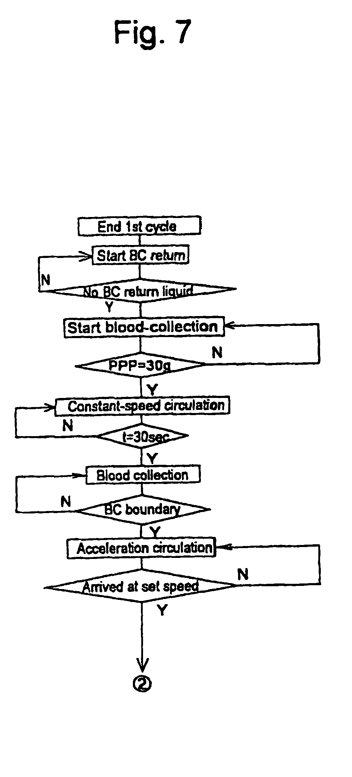

Then, the process proceeds to a second platelet collection operation, as shown in Fig. 7.

First, referring to Fig. 7, the controller 13 executes the buffy coat return step of returning the buffy coat collected in the first platelet collection step to the centrifugal separator 20 before the next blood collection step is executed. Once the process proceeds to the buffy coat return step, the controller 13 rotates the centrifugal separator 20 at a predetermined rotational speed (for example, 4800 rpm), opens the fifth flow path shutter means 85 and the fourth flow path shutter means 84, and starts the blood pump 11 at a predetermined rate (default value: 100 ml/min). The buffy coat contained in the buffy coat collection bag 27 is fed to the centrifugal separator 20 through the fifth flow path shutter means 85. The air in the centrifugal separator 20 is sent to the platelet collection bag 26 through the second line 22 and the fourth flow path shutter means 84. After the blood pump 11 rotates in an amount corresponding to the set collection amount of the buffy coat, the execution of the buffy coat return step is completed.

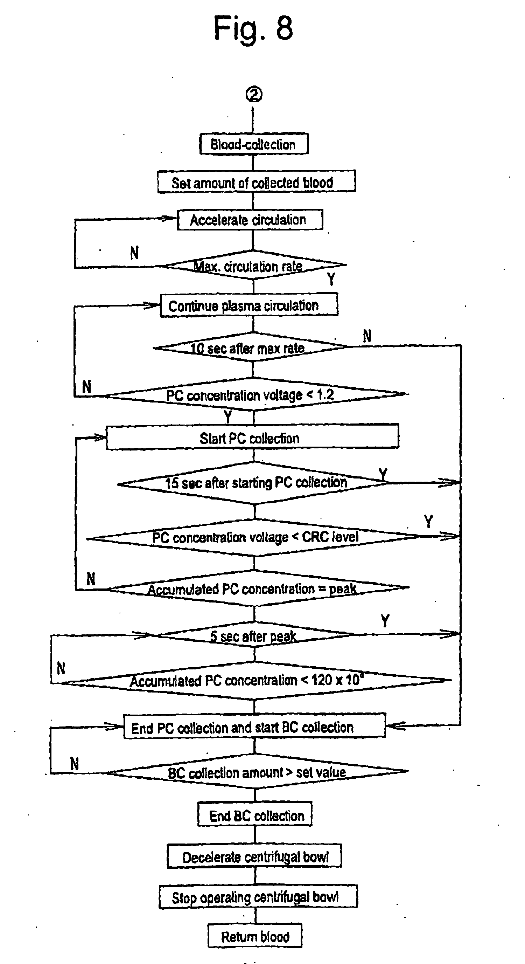

Then, the process proceeds to the program referred to as ② shown in Fig. 8 after termination of the execution of the first plasma collection step, the constant-speed circulation step, the second plasma collection step, and the acceleration circulation step. Then, the controller 13 executes the blood collection step for boundary adjustment, the platelet collection step, the buffy coat collection step, and the blood return step to complete the second platelet collection operation.

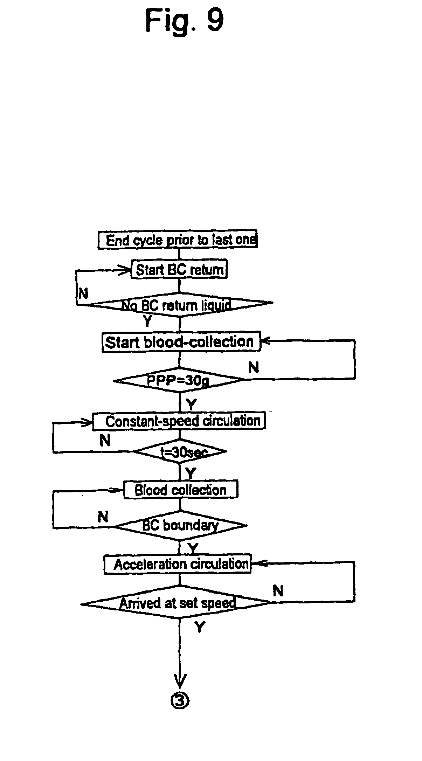

Now, explanation will be given on the last platelet collection operation as shown in Fig. 9. In this embodiment, the second operation is the last one. However, the third or subsequent platelet collection operation may be the last one as far as similar effects can be obtained. Each of these platelet collection operations except the last one is identical to the second platelet collection operation (Figs. 7 and 8).

First, as shown in Fig. 9, the buffy coat return step is executed. In this step, the buffy coat collected by the second platelet collection operation is returned to the centrifugal separator 20 prior to the execution of the next blood collection step.

When the process proceeds to the buffy coat return step, the controller 13 rotates the centrifugal separator 20 at a predetermined rotational speed (for example, 4800 rpm), opens the fifth flow path shutter means 85 and the fourth flow path shutter means 84, and activates the blood pump 11 at a predetermined rate (default value: 100 ml/min). The buffy coat contained in the buffy coat collection bag 27 passes through the fifth flow path shutter means 85 and is supplied to the centrifugal separator 20. The air in the centrifugal separator 20 is sent to the platelet collection bag 26 through the second line 22 and the fourth flow path shutter means 84. After the blood pump 11 rotates in an amount corresponding to the set collection amount of the buffy coat, the execution of the buffy coat return step terminates.

Then, the first plasma collection step is executed. That is, the first and second liquid supply pumps 11, 12 are started to collect the anticoagulant-added blood, and the centrifugal separator drive unit 10 is activated to collect a first predetermined amount of plasma from the blood and flow it into the plasma collection bag 25.

Upon start of the first blood collection, the first liquid supply pump 11 is rotated at a predetermined rate (for example, 60 ml/min). At this time, the second pump constituting the anticoagulant pump supplies the anticoagulant (such as the ACD-A solution) at the predetermined rate (for example, 1/10 of the blood pump rate). Blood supplied from the donor is mixed with the ACD-A solution. The ACD-A solution-added blood flows through the first line 21, passes through the chamber and the first flow path shutter means 81, and flows into the centrifugal separator 20. At this time, the sixth flow path shutter means 86, the fifth flow path shutter means 85, the second flow path shutter means 82, the third flow path shutter means 83, and the seventh flow path shutter means 87 are closed. Meanwhile the first flow path shutter means 81 and the fifth flow path shutter means 85 are opened. When the ACD-A solution-added blood is supplied to the centrifugal separator 20, the sterilized air that has been admitted into the centrifugal separator 20 flows into the buffy coat collection bag 27 through a line sensor and the fifth flow path shutter means 85. Simultaneously with the start of the blood collection step, the centrifugal separator 20 starts rotating at the predetermined speed (for example, 4800 rpm). The centrifugal separator 20 is supplied with the ACD-A solution-added blood during its rotation. Thus, the centrifugal separator 20 separates it centrifugally into the plasma layer (inner layer), the buffy coat layer (BC layer, intermediate layer), and the red cell layer (outer layer). When about 270 ml of the ACD-A solution-added blood exceeding the capacity of the centrifugal separator is supplied to the centrifugal separator 20, it is filled with the blood, and the plasma overflows through the outlet of the centrifugal separator 20. The turbidity sensor 14 mounted on the second line 22 connected to the outlet of the centrifugal separator 20 detects that the fluid flowing through the line has changed from the air to the plasma. Based on the detection signal outputted from the turbidity sensor 14, the controller 13 closes the fifth flow path shutter means 85 and opens the third flow path shutter means 83 to collect the plasma into the plasma collection bag 25. The weight of the plasma collection bag 25 is measured in advance by the weight sensor 16, and a signal indicating the measured weight is transmitted therefrom to the controller 13. Thus, when the weight of the plasma collected in the plasma collection bag 25 is increased to a predetermined amount (for example, 30 g), the controller 13 closes the first flow path shutter means 81 and opens the second flow path shutter means 82 to proceed to the constant-speed plasma circulation step.

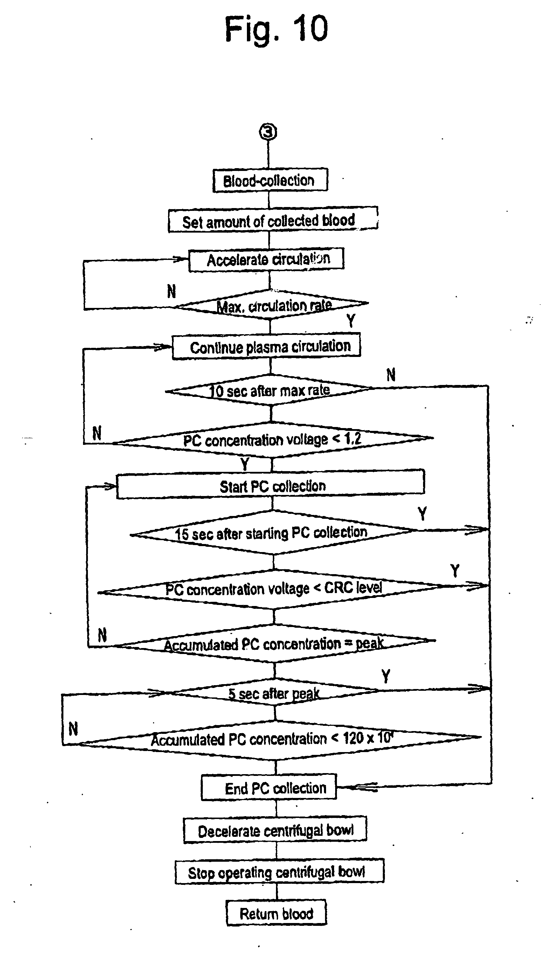

After the execution of the above-mentioned constant-speed circulation step, the second plasma circulation step, and the acceleration circulation step terminate, the process proceeds to the program referred to as ③ in Fig. 10 to execute the blood collection step for boundary adjustment, the platelet collection step, and the blood return step sequentially. Thereafter, the last platelet collection operation terminates. The last and second platelet collection operations are different from each other in that different air introduction bags are used in the constant-speed plasma circulation step of both operations and that the blood return step is executed without executing the buffy coat collection step in the last platelet collection operation.

It is known that in a particle suspension, as a particle concentration becomes higher, the apparent viscosity of the particle suspension becomes increasingly high. As the apparent viscosity becomes higher, the difference among sedimentation rates of particles having different specific gravities becomes increasingly high. When this fact is applied to the platelet collection step, it follows that as the apparent viscosity of the particle suspension in the centrifugal separator 20 becomes higher, the probability that the white cells having higher specific gravity than that of the platelets flow out together with the platelets becomes increasingly high. During the blood collection, the centrifugal separator 20 is rotating and a centrifugal force is constantly applied to the blood components in the centrifugal separator 20. Each blood component in the centrifugal separator 20, therefore, is progressively concentrated by the centrifugal force. Therefore, as shown in Fig. 3, the blood is separated into a red cell layer (outer layer), a buffy coat layer (intermediate layer), and a plasma layer (inner layer). The size of particles in each layer decreases sequentially in the direction from the outside of the centrifugal separator 20 to the inside thereof.

A plurality of what is called cake layers is formed in the centrifugal separator 20. The cake layers are gradually compressed. New whole blood flows into the centrifugal separator 20 from the cake layer formed of outermost blood cells and is trapped thereby. Meanwhile the other blood components pass through the cake layer and move to the buffy coat layer and the plasma layer. In the case where the blood cell cake layer and the buffy coat cake layer are excessively compressed, the gap between fine particles in each of the blood cell cake layer and the buffy coat cake layer is reduced. This leads to a phenomenon that platelets are trapped in the cell cake layer or the buffy coat cake layer. Thus, the collection efficiency of the platelets deteriorates.

According to the present invention, the plasma collection step is divided into a plurality of steps. Therefore, the time length of each plasma collection step can be shortened. Further, the cake layers are compressed very slowly by executing the acceleration plasma circulation step after the second blood collection terminates. In particular, because the acceleration plasma circulation step is executed, the cake layers are compressed very slowly and platelets embedded in a CRC layer (concentrated red cell layer) are moved upward even though the final circulation rate of plasma is set high. Thus, it is possible to collect the platelets into the BC layer. Because the BC layer is also moved upward, it is possible to accelerate separation and arrangement of the platelets and WBC (white cells) in the BC layer. Therefore, the platelets can be collected at improved collection efficiency.

### EXAMPLES

An apparatus having a circuit, as shown in Fig. 4, including two pumps of an ACD pump 12 and a blood pump 11 was prepared to compare the platelet collection performance of the example and that of a comparison example. Comparison tests were conducted on the same donor at an interval of two weeks or longer. Evaluated operation flows were compared as shown below (n = 2 examples). The number of obtained platelets contained in concentrated plasma and the number of white cells (WBC) were measured by Sysmex (R) NE-6000. The lower limit of the number of WBC which can be measured by the Sysmex NE-6000 was 0.1 x 10² [cells/muL]. Thus, Nageotte [1:9] method was used to measure the number of white cells of the samples that were lower than the lower limit. The collected amount of platelet was 40 ml.

Platelet collection operation was performed twice, using the above-described method. Blood collection rate was 60 ml/min. After the total amount of plasma reached 30 g, a first circulation was conducted under a condition of 200 ml/min for 30 seconds. After a BC boundary was detected, a second circulation was conducted under a condition of an initial rate of 60 ml/min for 30 seconds, an arrival rate of 200ml/min, an acceleration of 6.7ml/min/sec, and an accelerated time period of 21 seconds. The acceleration condition at a platelet collection time was 2.2ml/min/sec. The platelet collection rate was 200ml/min until the concentration of platelet attained a predetermined one. The buffy coat collection rate was 205 ml/min, 40 ml. In the second platelet collection operation, buffy coat was not collected.

### (Comparison Example)

The same apparatus as that used in the example was employed. The platelet collection operation was performed twice under the following conditions: The blood collection rate was 60 ml/min. After an optical sensor detected a buffy coat layer, a first circulation was conducted at the rate of 200 ml/min for 3 seconds, and a second circulation was conducted at the rate of 200 ml/min for 20 seconds. Platelets were collected at an acceleration rate of 8 ml/min/sec until the concentration of platelet attained 50 % of the maximum concentration.

The concentration of platelets contained in concentrated plasma and the concentration of white cells in the example and the comparison example are shown as follows:

**Table 1 Platelet concentration [cells/muL]**

| | Sample 1 | Sample 2 |
|---|---|---|
| Example | 113.8 x 10⁴ | 126.5 x 10⁴ |
| Comparison Ex. | 104.4 x 10⁴ | 112.4 x 10⁴ |

**Table 2 White cell concentration [cells/muL]**

| | Sample 1 | Sample 2 |
|---|---|---|
| Example | 0.1 x 10¹ | 0.1x10² |
| Comparison Ex. | 0.4 x10² | 0.6 x10² |

The blood component collection apparatus according to the present invention has two pumps. Thus, it can be reduced in size. A blood collection is temporarily suspended in a given platelet collection operation. The plasma re-circulation step for re-circulating collected plasma to the centrifugal separator is executed at least twice. Further, the plasma re-circulation step is executed as the acceleration circulation in a later time. Therefore, it is possible to prevent a blood cell layer and a buffy coat layer from being excessively compressed in the centrifugal separator, move upward platelets embedded in a concentrated red cell layer, and collect platelets into the buffy coat layer. Because the buffy coat layer also moves upward, it is possible to accelerate separation and arrangement of the platelets and white cells in the buffy coat layer. Therefore, the blood component collection apparatus produces a high-concentration platelet-containing solution (plasma containing concentrated platelets) that contains a small number of white cells at a high platelet collection efficiency.

## Claims

1. A blood component collection apparatus comprising
a blood component collection circuit (2) including:
a centrifugal separator (20) having a rotor (142), an internal blood storage space, an inlet and an outlet communicating with said blood storage space and centrifugally separating blood introduced thereinto through said inlet by a rotation of said rotor;
a first line (21) for connecting a connecting portion of a blood collection needle (29) or a blood collection instrument and said inlet of said centrifugal separator with each other;
a second line (22) connected to said outlet of said centrifugal separator;
a third line (23) connected to said first line, for injecting an anticoagulant to said blood;
a plasma collection bag (25) having a first tube connected with said first line and a second tube connected with said second line; and
a platelet collection bag (26) connected with said second line;
said blood component collection apparatus further comprising
a centrifugal separator drive unit (10) for rotating said rotor of said centrifugal separator;
a first liquid supply pump (11) which is used for said first line and located at a position of said first line between said centrifugal separator and a connecting portion for connecting said first tube to said first line;
a second liquid supply pump (12) which is used for said third line;
a plurality of flow path shutter means (81-86) for opening/closing flow paths of said blood component collection circuit; and
a controller (13) for controlling said centrifugal separator drive unit, said first liquid supply pump, said second liquid supply pump, and a plurality of said flow path shutter means;
wherein said controller (13) is configured to control said centrifugal separator drive unit, said first liquid supply pump, said second liquid supply pump, and a plurality of said flow path shutter means to execute a platelet collection operation including:
a plasma collection/constant-speed circulation step including a first plasma collection step of collecting anticoagulant-added blood by starting said first and second liquid supply pumps (11, 12) and collecting a predetermined amount of plasma into said plasma collection bag (25) by activating said centrifugal separator drive unit (10); and a constant-speed plasma circulation step which is executed after said execution of said first plasma collection step terminates to temporarily suspend a blood collection and circulate said plasma contained in said plasma collection bag to said centrifugal separator (20) at a constant speed by driving said first liquid supply pump (11),
a plasma collection/acceleration circulation step including a second plasma collection step which is executed after said execution of said plasma collection/constant-speed circulation step terminates to collect said anticoagulant-added blood by starting said first and second liquid supply pumps (11, 12) and collect said plasma by activating said centrifugal separator drive unit (10); and an acceleration plasma circulation step which is executed after said execution of said second plasma collection step terminates to temporarily suspend said blood collection and circulate said plasma contained in said plasma collection bag to said centrifugal separator at an accelerating speed by driving said first liquid supply pump (11),
a platelet collection step which is executed after said execution of said plasma collection/acceleration circulation step terminates to flow out platelets from said centrifugal separator (20) and collect said platelets into said platelet collection bag (26) by accelerating a plasma circulation rate by means of said first liquid supply pump, and
a blood return step which is executed after said execution of said platelet collection step terminates to return said blood inside said centrifugal separator to a donor.

2. A blood component collection apparatus according to claim 1, wherein after said plasma collection/acceleration circulation step terminates and before said plasma circulation rate is accelerated by means of said first liquid supply pump (11), said controller (13) executes said first plasma collection step of collecting said anticoagulant-added blood by starting said first and second liquid supply pumps and collecting said predetermined amount of said plasma into said plasma collection bag (25) by activating said centrifugal separator drive unit.

3. A blood component collection apparatus according to claim 1 or 2, further comprising a weight sensor (10) for measuring a weight of said plasma collection bag.

4. A blood component collection apparatus according to any one of claims 1 to 3, wherein said controller (13) is configured to control said centrifugal separator drive unit (10), said first liquid supply pump (11), said second liquid supply pump (12), and a plurality of said flow path shutter means (81-86) to execute said platelet collection operation at least twice.

5. A blood component collection apparatus according to any one of claims 1 to 4, wherein said blood component collection circuit has a buffy coat collection bag (27) connected with said second line (22); and after said execution of said platelet collection step terminates and before said execution of said blood return step starts, said controller executes a buffy coat collection step of flowing out a buffy coat from said centrifugal separator and collecting said buffy coat into said buffy coat collection bag by setting a plasma circulation rate higher than a final plasma circulation rate in said platelet collection step by means of said first liquid supply pump (11).

6. A blood component collection apparatus according to any one of claims 1 to 4, wherein said blood component collection circuit has a buffy coat collection bag (27) connected with said second line (22); and after said execution of said platelet collection step terminates and before said execution of said blood return step starts, said controller executes a buffy coat collection step of flowing out a buffy coat from said centrifugal separator and collecting said buffy coat into said buffy coat collection bag by setting said plasma circulation rate equally to or higher than said final plasma circulation rate in said platelet collection step by means of said first liquid supply pump (11) and a rotor rate of said centrifugal separator equally to or lower than a rotor rate of said centrifugal separator in said platelet collection step.

7. A blood component collection apparatus according to claim 5 or 6, wherein when a platelet collection operation is executed after said buffy coat collection step terminates, said controller (13) controls said first liquid supply pump (11) and a plurality of said flow path shutter means to execute a buffy coat return step of returning said collected buffy coat to said centrifugal separator before a subsequent first plasma collection step is executed.

## Revendications

1. Dispositif de collecte de composants sanguins comprenant :
un circuit de collecte de composants sanguins (2) comportant :
un séparateur centrifuge (20) comportant un rotor (142), un espace interne de stockage de sang, une entrée et une sortie communiquant avec ledit espace de stockage de sang, et séparant de façon centrifuge, par rotation dudit rotor, les composants du sang qui y est introduit par ladite entrée ;
une première conduite (21) pour relier une partie de connexion d'une aiguille de collecte de sang (29) ou un instrument de collecte de sang et ladite entrée dudit séparateur centrifuge ;
une deuxième conduite (22) connectée à ladite sortie dudit séparateur centrifuge ;
une troisième conduite (23) connectée à ladite première conduite, pour injecter un anticoagulant dans ledit sang ;
une poche de collecte de plasma (25) ayant un premier tube relié à ladite première conduite et un deuxième tube relié à ladite deuxième conduite ; et
une poche de collecte de plaquettes (26) reliée à ladite deuxième conduite ;
ledit dispositif de collecte de composants sanguins comprenant en outre :
une unité d'entraînement (10) de séparateur centrifuge pour faire tourner ledit rotor dudit séparateur centrifuge ;
une première pompe d'alimentation en liquide (11) qui est utilisée pour ladite première conduite et située en une position de ladite première conduite entre ledit séparateur centrifuge et une partie de connexion servant à connecter ledit premier tube à ladite première conduite ;
une deuxième pompe d'alimentation en liquide (12) qui est utilisée pour ladite troisième conduite ;
une pluralité de moyens d'obturation de chemins d'écoulement (81-86) pour ouvrir et fermer les chemins d'écoulement dudit circuit de collecte de composants sanguins ; et
un dispositif de commande (13) pour commander ladite unité d'entraînement de séparateur centrifuge, ladite première pompe d'alimentation en liquide, ladite deuxième pompe d'alimentation en liquide et une pluralité desdits moyens d'obturation de chemins d'écoulement ;
dans lequel ledit dispositif de commande (13) est configuré pour commander ladite unité d'entraînement de séparateur centrifuge, ladite première pompe d'alimentation en liquide, ladite deuxième pompe d'alimentation en liquide et une pluralité desdits moyens d'obturation de chemins d'écoulement pour exécuter une opération de collecte de plaquettes comprenant :
une étape de collecte de plasma/circulation à vitesse constante incluant une première étape de collecte de plasma qui consiste à collecter du sang contenant de l'anticoagulant en démarrant lesdites première et deuxième pompes d'alimentation en liquide (11, 12) et en collectant une quantité prédéterminée de plasma dans ladite poche de collecte de plasma (25) en activant ladite unité d'entraînement (10) de séparateur centrifuge, et une étape de circulation de plasma à vitesse constante qui est exécutée après la fin de ladite exécution de ladite première étape de collecte de plasma pour suspendre temporairement une collecte de sang et faire circuler ledit plasma contenu dans ladite poche de collecte de plasma vers ledit séparateur centrifuge (20) à une vitesse constante en entraînant ladite première pompe d'alimentation en liquide (11),
une étape de collecte de plasma/circulation accélérée incluant une deuxième étape de collecte de plasma qui est exécutée après la fin de ladite exécution de ladite étape de collecte de plasma/circulation à vitesse constante pour collecter ledit sang contenant de l'anticoagulant en démarrant lesdites première et deuxième pompes d'alimentation en liquide (11, 12) et collecter ledit plasma en activant ladite unité d'entraînement (10) de séparateur centrifuge, et une étape de circulation de plasma accélérée qui est exécutée après la fin de ladite exécution de ladite deuxième étape de collecte de plasma pour suspendre temporairement ladite collecte de sang et faire circuler ledit plasma contenu dans ladite poche de collecte de plasma vers ledit séparateur centrifuge à une vitesse croissante en entraînant ladite première pompe d'alimentation en liquide (11),
une étape de collecte de plaquettes qui est exécutée après la fin de ladite exécution de ladite étape de collecte de plasma/circulation accélérée pour faire sortir les plaquettes du séparateur centrifuge (20) et collecter lesdites plaquettes dans ladite poche de collecte de plaquettes (26) en augmentant la vitesse de circulation du plasma au moyen de ladite première pompe d'alimentation en liquide (11), et
une étape de renvoi du sang qui est exécutée après la fin de ladite exécution de ladite étape de collecte de plaquettes pour renvoyer le sang qui se trouve à l'intérieur dudit séparateur centrifuge à un donneur.

2. Dispositif de collecte de composants sanguins selon la revendication 1, dans lequel après la fin de ladite étape de collecte de plasma/ circulation accélérée et avant que ladite vitesse de circulation du plasma soit augmentée au moyen de ladite première pompe d'alimentation en liquide (11), ledit dispositif de commande (13) exécute ladite première étape de collecte de plasma qui consiste à collecter ledit sang contenant de l'anticoagulant en démarrant lesdites première et deuxième pompes d'alimentation en liquide et en collectant ladite quantité prédéterminée de plasma dans ladite poche de collecte de plasma (25) en activant ladite unité d'entraînement de séparateur centrifuge.

3. Dispositif de collecte de composants sanguins selon la revendication 1 ou 2, comprenant en outre un capteur de poids (16) pour mesurer une masse de ladite poche de collecte de plasma.

4. Dispositif de collecte de composants sanguins selon l'une quelconque des revendications 1 à 3, dans lequel ledit dispositif de commande (13) est configuré pour commander ladite unité d'entraînement (10) de séparateur centrifuge, ladite première pompe d'alimentation en liquide (11), ladite deuxième pompe d'alimentation en liquide (12) et une pluralité desdits moyens d'obturation de chemins d'écoulement (81-86) pour exécuter ladite opération de collecte de plaquettes au moins deux fois.

5. Dispositif de collecte de composants sanguins selon l'une quelconque des revendications 1 à 4, dans lequel ledit circuit de collecte de composants sanguins comporte une poche de collecte de couche leucocytaire (27) connectée à ladite deuxième conduite (22), et après la fin de ladite exécution de ladite étape de collecte de plaquettes et avant le début de ladite exécution de ladite étape de renvoi du sang, ledit dispositif de commande exécute une étape de collecte de couche leucocytaire qui consiste à faire sortir une couche leucocytaire dudit séparateur centrifuge et à collecter ladite couche leucocytaire dans ladite poche de collecte de couche leucocytaire en réglant une vitesse de circulation de plasma sur une vitesse supérieure à une vitesse de circulation de plasma finale dans ladite étape de collecte de plaquettes au moyen de ladite première pompe d'alimentation en liquide (11).

6. Dispositif de collecte de composants sanguins selon l'une quelconque des revendications 1 à 4, dans lequel ledit circuit de collecte de composants sanguins comporte une poche de collecte de couche leucocytaire (27) connectée à ladite deuxième conduite (22), et après la fin de ladite exécution de ladite étape de collecte de plaquettes et avant le début de ladite exécution de ladite étape de renvoi du sang, ledit dispositif de commande exécute une étape de collecte de couche leucocytaire qui consiste à faire sortir une couche leucocytaire dudit séparateur centrifuge et à collecter ladite couche leucocytaire dans ladite poche de collecte de couche leucocytaire en réglant ladite vitesse de circulation de plasma sur une vitesse supérieure ou égale à ladite vitesse de circulation de plasma finale dans ladite étape de collecte de plaquettes au moyen de ladite première pompe d'alimentation en liquide (11) et une vitesse de rotor dudit séparateur centrifuge sur une vitesse inférieure ou égale à une vitesse de rotor dudit séparateur centrifuge dans ladite étape de collecte de plaquettes.

7. Dispositif de collecte de composants sanguins selon la revendication 5 ou 6, dans lequel, lorsqu'une opération de collecte de plaquettes est exécutée après la fin de ladite étape de collecte de couche leucocytaire, ledit dispositif de commande (13) commande ladite première pompe d'alimentation en liquide (11) et une pluralité desdits moyens d'obturation de chemins d'écoulement pour exécuter une étape de renvoi de couche leucocytaire qui consiste à renvoyer ladite couche leucocytaire collectée vers ledit séparateur centrifuge avant qu'une première étape de collecte de plasma suivante ne soit exécutée.

## Patentansprüche

1. Vorrichtung zur Sammlung von Blutbestandteilen, die wie folgt aufweist
einen Kreis (2) zur Sammlung von Blutbestandteilen, der Folgendes umfasst:
einen Zentrifugalseparator (20), welcher einen Rotor (142) hat, einen internen Blutspeicherraum, einen Einlass und einen Auslass, die mit dem Blutspeicherraum in Verbindung stehen, und welcher durch den Einlass in denselben eingelassenes Blut mittels Rotation des Rotors zentrifugal trennt;
eine erste Leitung (21) zur Verbindung eines Verbindungsabschnitts einer Blutentnahmenadel (29) oder eines Blutentnahmeinstruments und des Einlasses des Zentrifugalseparators miteinander;
eine mit dem Auslass des Zentrifugalseparators verbundene zweite Leitung (22);
eine mit der ersten Leitung verbundene dritte Leitung (23), um ein Antikoagulans in das Blut zu injizieren;
einen Plasmasammelbeutel (25) mit einer ersten Röhre, die mit der ersten Leitung verbunden ist, und einer zweiten Röhre, die mit der zweiten Leitung verbunden ist; und
einen Blutplättchensammelbeutel (26), der mit der zweiten Leitung verbunden ist;
die Vorrichtung zur Sammlung von Blutbestandteilen weiterhin umfassend:
eine Zentrifugalseparatorantriebseinheit (10), um den Rotor des Zentrifugalseparators zu drehen;
eine erste Flüssigkeitszuführpumpe (11), die für die erste Leitung eingesetzt wird und an einer Stelle der ersten Leitung zwischen dem Zentrifugalseparator und einem Verbindungsabschnitt zur Verbindung der ersten Röhre mit der ersten Leitung liegt;
eine zweite Flüssigkeitszuführpumpe (12), die für die dritte Leitung eingesetzt wird;
eine Mehrzahl von Durchflusswegverschlussmitteln (81-86) zum Öffnen/Schließen von Durchflusswegen des Kreises zur Sammlung von Blutbestandteilen; und
eine Steuerungseinheit (13) zur Steuerung der Zentrifugalseparatorantriebseinheit, der ersten Flüssigkeitszuführpumpe, der zweiten Flüssigkeitszuführpumpe, und einer Mehrzahl der Durchflusswegverschlussmittel;
wobei die Steuerungseinheit (13) gestaltet ist, um die Zentrifugalseparatorantriebseinheit, die erste Flüssigkeitszuführpumpe, die zweite Flüssigkeitszuführpumpe, und eine Mehrzahl der Durchflusswegverschlussmittel zu steuern zwecks Durchführung eines Blutplättchensammelvorgangs umfassend:
einen Plasmasammel-/Konstantgeschwindigkeitszirkulationsschritt umfassend einen ersten Plasmasammelschritt bestehend im Sammeln von Blut mit zugefügtem Antikoagulans durch Starten der ersten und zweiten Flüssigkeitszuführpumpe (11, 12) und im Sammeln einer vorgegebenen Menge Plasma in dem Plasmasammelbeutel (25) durch in Betrieb Setzen der Zentrifugalseparatorantriebseinheit (10); und einen Konstantgeschwindigkeitsplasmazirkulationsschritt, der erfolgt, nachdem die Durchführung des ersten Plasmasammelschritts beendet ist, um zeitweilig eine Blutsammlung einzustellen und das in dem Plasmasammelbeutel enthaltene Plasma zu dem Zentrifugalseparator (20) mit einer konstanten Geschwindigkeit durch Antreiben der ersten Flüssigkeitszuführpumpe (11) zirkulieren zu lassen,
einen Plasmasammel-/Beschleunigungszirkulationsschritt umfassend einen zweiten Plasmasammelschritt, der erfolgt, nachdem die Durchführung des Plasmasammel/Konstantgeschwindigkeitszirkulationsschritts beendet ist, um das Blut mit zugefügtem Antikoagulans durch Starten der ersten und zweiten Flüssigkeitszuführpumpe (11, 12) zu sammeln und um das Plasma durch in Betrieb Setzen der Zentrifugalseparatorantriebseinheit (10) zu sammeln; und einen Beschleunigungsplasmazirkulationsschritt, der erfolgt, nachdem die Durchführung des zweiten Plasmasammelschritts beendet ist, um zeitweilig die Blutsammlung einzustellen und das in dem Plasmasammelbeutel enthaltene Plasma zu dem Zentrifugalseparator mit einer sich beschleunigenden Geschwindigkeit durch Antreiben der ersten Flüssigkeitszuführpumpe (11) zirkulieren zu lassen,
einen Blutplättchensammelschritt, der erfolgt, nachdem die Durchführung des Plasmasammel-/Beschleunigungszirkulationsschritts beendet ist, um Blutplättchen aus dem Zentrifugalseparator (20) herausfließen zu lassen und die Blutplättchen in dem Blutplättchensammelbeutel (26) zu sammeln durch Beschleunigen einer Plasmazirkulationsrate mittels der ersten Flüssigkeitszuführpumpe, und
einen Blutrückgabeschritt, der erfolgt, nachdem die Durchführung des Blutplättchensammelschritts beendet ist, um das Blut im Innern des Zentrifugalseparators an einen Spender zurückzugeben.

2. Vorrichtung zur Sammlung von Blutbestandteilen nach Anspruch 1, wobei, nach Beendigung des Plasmasammel-/Beschleunigungszirkulationsschritts und vor Beschleunigung der Plasmazirkulationsrate mittels der ersten Flüssigkeitszuführpumpe (11), die Steuerungseinheit (13) den ersten Plasmasammelschritt ausführt bestehend im Sammeln des Bluts mit zugefügtem Antikoagulans durch Starten der ersten und zweiten Flüssigkeitszuführpumpe und im Sammeln der vorgegebenen Menge des Plasmas in dem Plasmasammelbeutel (25) durch in Betrieb Setzen der Zentrifugalseparatorantriebseinheit.

3. Vorrichtung zur Sammlung von Blutbestandteilen nach Anspruch 1 oder 2, die weiterhin einen Gewichtssensor (16) zur Messung eines Gewichts des Plasmasammelbeutels aufweist.

4. Vorrichtung zur Sammlung von Blutbestandteilen nach einem der Ansprüche 1 bis 3, wobei die Steuerungseinheit (13) gestaltet ist, um die Zentrifugalseparatorantriebseinheit (10), die erste Flüssigkeitszuführpumpe (11), die zweite Flüssigkeitszuführpumpe (12), und eine Mehrzahl der Durchflusswegverschlussmittel (81-86) zu steuern, damit der Blutplättchensammelvorgang mindestens zweimal durchgeführt wird.

5. Vorrichtung zur Sammlung von Blutbestandteilen nach einem der Ansprüche 1 bis 4, wobei der Kreis zur Sammlung von Blutbestandteilen einen Leukozytenfilmsammelbeutel (27) aufweist, der mit der zweiten Leitung (22) verbunden ist; und, nach Beendigung der Durchführung des Blutplättchensammelschritts und vor Beginn der Durchführung des Blutrückgabeschritts, die Steuerungseinheit einen Leukozytenfilmsammelschritt ausführt bestehend im Herausfließenlassen eines Leukozytenfilms aus dem Zentrifugalseparator und im Sammeln des Leukozytenfilms in dem Leukozytenfilmsammelbeutel, indem sie eine Plasmazirkulationsrate höher einstellt als eine finale Plasmazirkulationsrate in dem Blutplättchensammelschritt mittels der ersten Flüssigkeitszuführpumpe (11).

6. Vorrichtung zur Sammlung von Blutbestandteilen nach einem der Ansprüche 1 bis 4, wobei der Kreis zur Sammlung von Blutbestandteilen einen Leukozytenfilmsammelbeutel (27) aufweist, der mit der zweiten Leitung (22) verbunden ist; und, nach Beendigung der Durchführung des Blutplättchensammelschritts und vor Beginn der Durchführung des Blutrückgabeschritts, die Steuerungseinheit einen Leukozytenfilmsammelschritt ausführt bestehend im Herausfließenlassen eines Leukozytenfilms aus dem Zentrifugalseparator und im Sammeln des Leukozytenfilms in dem Leukozytenfilmsammelbeutel, indem sie die Plasmazirkulationsrate genauso hoch wie oder höher als die finale Plasmazirkulationsrate in dem Blutplättchensammelschritt mittels der ersten Flüssigkeitszuführpumpe (11) und eine Rotorgeschwindigkeit des Zentrifugalseparators genauso hoch wie oder niedriger als eine Rotorgeschwindigkeit des Zentrifugalseparators in dem Blutplättchensammelschritt einstellt.

7. Vorrichtung zur Sammlung von Blutbestandteilen nach Anspruch 5 oder 6, wobei, wenn ein Blutplättchensammelvorgang durchgeführt wird, nachdem der Leukozytenfilmsammelschritt beendet ist, die Steuerungseinheit (13) die erste Flüssigkeitszuführpumpe (11) und eine Mehrzahl der Durchflusswegverschlussmittel steuert, um einen Leukozytenfilmrückgabeschritt durchzuführen bestehend im Zurückgeben des gesammelten Leukozytenfilms an den Zentrifugalseparator, bevor ein anschließender erster Plasmasammelschritt erfolgt.
